# EUROPEAN PATENT SPECIFICATION

(11) **EP 4 252 744 B1**
(45) Date of publication and mention of the grant of the patent: **18.02.2026**
(21) Application number: 21898185.0
(22) Date of filing: 30.11.2021
(51) Int. Cl.: A61K 9/14, A61K 47/02, A61K 47/10, A61K 35/20, A23L 33/10

(54) **SOLID COMPOSITION**
FESTE ZUSAMMENSETZUNG
COMPOSITION SOLIDE

(30) Priority: 30.11.2020 JP 2020198592
(43) Date of publication of application: 04.10.2023
(73) Proprietor: Kao Corporation, Chuo-ku Tokyo 103-8210 (JP)
(72) Inventor: SHIBATA, Ako, Tokyo 131-8501 (JP)
(74) Representative: Hoffmann Eitle
(86) International application number: PCT/JP2021/043794
(87) International publication number: WO 2022/114214

(56) References cited:
- WO-A1-2008/093657
- WO-A1-2015/198480
- WO-A1-2015/198480
- JP-A- 2009 221 157
- JP-A- 2017 212 961
- JP-A- 2018 030 789
- JP-A- 2018 030 789
- JP-A- 2018 085 965
- JP-A- 2019 089 746
- JP-B2- 6 652 366

## Description

### FIELD OF THE INVENTION

The present invention relates to a solid composition.

### BACKGROUND OF THE INVENTION

Sphingomyelin is one of the typical sphingophospholipids widely distributed in the animal body, and has a structure in which phosphocholine is attached to a ceramide skeleton composed of a sphingoid base and a fatty acid. Sphingomyelin is also contained in cow milk, and about one-third of phospholipids, which account for about 1% of the total lipids, are sphingomyelin. It is known that phospholipids in cow milk are mainly present in milk fat globule membranes.

For milk-derived sphingomyelins, researches on and its physiological function have been progressed in recent years. For example, milk-derived sphingomyelin has been reported to have an improving effect of learning ability (Patent Literature 1), an improving effect of motor function (Patent Literature 2), and a skin-beautifying effect (Patent Literature 3).

In order to effectively obtain a physiological function of sphingomyelin, it is desirable to form a solid composition such as a tablet or a granule which can be easily and effortlessly ingested for a long period of time. For example, Patent Literature 4 discloses a solid composition containing milk-derived sphingomyelins and sugar alcohols. Patent Literature 5 discloses a composition such as tablet containing casein hydrolysate and milk-derived ceramides.

JP 2018 030789 A describes a solid composition that contains the following components (A) and (B): (A) milk fat globule membrane components of 20-60 mass%, and (B) silicon dioxide of 0.5-5 mass%.

JP 6 652366 B2 describes a milk fat globule membrane component-containing beverage composition that comprises a milk fat globule membrane component (A), the milk fat globule membrane component comprising (a) sphingomyelin 0.02-0.5 mass%. The beverage composition also comprises (B) soybean polysaccharide component in an amount 3.5-27 times the mass of (a), (C) crystalline cellulose component in an amount 1.3-20 times the mass of (a), and (D) water.

| | |
|---|---|
| (Patent Literature 1) | JP-A-2007-246404 |
| (Patent Literature 2) | JP-A-2011-157330 |
| (Patent Literature 3) | JP-A-2008-184428 |
| (Patent Literature 4) | WO-A-2015/198480 |
| (Patent Literature 5) | JP-A-2009-221157 |

### SUMMARY OF THE INVENTION

The present invention provides a solid composition comprising the following components (A), (B) and (C):
(A) 0.15 mass% or more and 3.0 mass% or less of a milk-derived sphingomyelin;
(B) a sugar alcohol; and
(C) potassium;
wherein a mass ratio of the component (C) to the component (A), [(C)/(A)], is from 0.2 to 9.0.

### DETAILED DESCRIPTION OF THE INVENTION

However, the present inventor found that, when a solid composition containing a milk-derived sphingomyelin is stored for a long period of time, not only the milk flavor of the milk-derived sphingomyelin is attenuated over time, but also a deteriorated flavor different from the milk flavor is generated, and the flavor after storage of the overall solid composition is weakened.

Accordingly, the present invention relates to providing a solid composition comprising a milk-derived sphingomyelin with suppressed flavor deterioration associated with storage.

As a result of earnest research, the present inventors found that the milk flavor of a milk-derived sphingomyelin can be retained even after storage by combining sugar alcohol with a milk-derived sphingomyelin and further potassium at a predetermined ratio, and the occurrence of deteriorated flavor can be suppressed.

The present invention provides a solid composition containing a milk-derived sphingomyelin, providing a milk flavor of the milk-derived sphingomyelin even after storage, and as well providing an excellent flavor.

The solid composition of the present invention comprises (A) a milk-derived sphingomyelin. The milk-derived sphingomyelin may be derived from any one of cow milk (including raw milk), goat milk, and the like. Among them, sphingomyelin derived from cow milk is preferred from the viewpoint of abundant food experience ever, low cost, and imparting an excellent milk flavor.

As the fatty acid constituting the milk-derived sphingomyelin, a saturated or unsaturated linear fatty acid is preferable. The number of carbon atoms of the linear fatty acid is preferably from 12 to 30, more preferably from 14 to 26.

In the milk-derived sphingomyelin, the proportion of the fatty acid having 16 or more carbon atoms in the total fatty acid constituting the milk-derived sphingomyelin is preferably 50 mass% or more, more preferably 60 mass% or more, further more preferably 70 mass% or more, and further more preferably 80 mass% or more, from the viewpoint of imparting good milk flavor. It may also be 100 mass%.

The (A) milk-derived sphingomyelin may be one which has been purified, but it is preferable to use the milk-derived sphingomyelin in the form of a milk-derived sphingomyelin inclusion containing milk-derived sphingomyelin without intentional purification, from the viewpoint of improving the milk flavor after storage.

For example, butter milk, which remains after granular butter is removed from cream obtained by centrifuging cow milk and the like, butter serum, which remains after butter oil and anhydrous milk fat (AMF) are removed from cream in the same manner, milk fat globule membranes obtained by concentrating these by a known method such as membrane treatment, whey protein concentrate (WPC) obtained as a residue obtained by coagulating cow milk and the like contain a large amount of sphingomyelin, and therefore it is preferable to use them as they are. In addition, these may be purified by a known method such as dialysis, ammonium sulfate fractionation, gel-filtration, isoelectric point precipitation, ionexchange chromatography, and solvent-fractionation to improve the purity of sphingomyelin (Sanchez-Juanes, Int Dairy J, 273, 2009).

Commercially available sphingomyelin can be used.

As the milk-derived sphingomyelin, Nippon Oil Co., Ltd. "COATSOME NM-70", Nagara Science Co., Ltd. "Sphingomyelin, from Milk" and the like are commercially available.

The purity of the milk-derived sphingomyelin in the milk-derived sphingomyelin inclusion is not particularly limited, and may be any purity as long as the desired physiological effect can be exerted when the milk-derived sphingomyelin is formulated as a solid composition. The purity is preferably 0.2 mass% or more, more preferably 0.4 mass% or more, and further more preferably 1.0 mass% or more, from the viewpoint of providing an intake form facilitating a small amount at one time, and is preferably 100 mass% or less, more preferably 50 mass% or less, further more preferably 30 mass% or less, and further more preferably 25% or less from the viewpoint of flavor and handling.

The content of the lipid in the milk-derived sphingomyelin inclusion is preferably 2 mass% or more, more preferably 4 mass% or more, and further more preferably 10 mass% or more from the viewpoint of physiological effects, and is preferably 100 mass% or less, more preferably 90 mass% or less, and further more preferably 60 mass% or less from the viewpoint of flavor and handling.

The content of phospholipids in the milk-derived sphingomyelin inclusion is preferably 0.5 mass% or more, more preferably 1 mass% or more, further more preferably 2 mass% or more, and further more preferably 3 mass% or more from the viewpoint of physiological function, and is preferably 100 mass% or less, more preferably 85 mass% or less, further more preferably 70 mass% or less, and further more preferably 60% or less from the viewpoint of flavor and handling.

In the present specification, the contents of milk-derived sphingomyelin, lipid, and phospholipid in the milk-derived sphingomyelin inclusion represents a mass ratio of those in relation to a dried milk-derived sphingomyelin inclusion.

The content of the (A) milk-derived sphingomyelin in the solid composition of the present invention is 0.15 mass% or more and 3.0 mass% or less. From the viewpoint of imparting excellent milk flavor at an initial timing and after storage, ensuring an intake amount to exert an effective a physiological effect, and providing an intake form facilitating a small amount at one time, the content of the (A) milk-derived sphingomyelin in the solid composition is 0.15 mass% or more, preferably 0.2 mass% or more, more preferably 0.3 mass% or more, further more preferably 0.5 mass% or more, even more preferably 0.8 mass% or more; and from the viewpoint of imparting an excellent milk flavor after storage and suppressing occurrence of deteriorated flavor, the content of the component (A) in the solid composition is less than 3.0 mass%, preferably less than 2.73 mass%, and more preferably less than 2.11 mass% or less. The content of the (A) milk-derived sphingomyelin in the solid composition is 0.15 mass% or more and 3.0 mass% or less, preferably 0.2 mass% or more and 3.0 mass% or less, more preferably 0.3 mass% or more and 3.0 mass% or less, further more preferably 0.5 mass% or more and 2.73 mass% or less, even more preferably 0.8 mass% or more and 2.11 mass% or less.

As used herein, analysis of the (A) milk-derived sphingomyelin will follow the method described in the Examples hereinafter.

The solid composition of the present invention comprises (B) a sugar alcohol. The sugar alcohol is not particularly limited, and examples thereof include maltitol, erythritol, xylitol, sorbitol, mannitol, lactitol, trehalose, and reduced palatinose. Among them, maltitol, erythritol and xylitol are preferable, and maltitol is more preferable from the viewpoint of imparting excellent milk flavor at initial timing and after storage. These may be used alone or used in combination of two or more. The sugar alcohol may be anhydrous or hydrated.

The content of the (B) sugar alcohol in the solid composition of the present invention can be appropriately adjusted in a range as long as it does not impair the objective of the present invention. The content of the (B) sugar alcohol is preferably 1 mass% or more, more preferably 5 mass% or more, from the viewpoint of imparting an excellent milk flavor at initial timing and after storage, and suppressing occurrence of deteriorated flavor. In addition, the content of the (B) sugar alcohol is preferably 70 mass% or less, more preferably 50 mass% or less, and further more preferably 30 mass% or less, from the viewpoint of suppressing occurrence of deteriorated flavor of the overall solid composition by the sweetness derived from the sugar alcohol. The content of the (B) sugar alcohol in the solid composition is preferably 1 mass% or more and 70 mass% or less, more preferably 5 mass% or more and 50 mass% or less, and further more preferably 5 mass% or more and 30 mass% or less.

The (B) sugar alcohol can be analyzed by high performance liquid chromatography (water extraction).

In the solid composition of the present invention, a mass ratio of the component (B) to the component (A), [(B)/(A)], is preferably 3 or more, more preferably 5 or more, and even more preferably 8 or more from the viewpoint of imparting an excellent milk flavor at the initial timing and after storage and suppressing occurrence of deteriorated flavor. In addition, from the viewpoint of suppressing occurrence of deterioration of the flavor of the overall solid composition by a sweet taste derived from the sugar alcohol, it is preferably 70 or less, more preferably 50 or less, and further more preferably 30 or less. The mass ratio of the component (B) to the component (A), [(B)/(A)], is preferably 3 or more and 70 or less, more preferably 5 or more and 50 or less, even more preferably 8 or more and 30 or less.

The solid composition of the present invention comprises (C) potassium.

The source of potassium is not particularly limited, and examples thereof include inorganic salts such as potassium chloride, potassium sulfate, potassium nitrate, and potassium carbonate, and organic salts such as potassium acetate, potassium citrate, and potassium tartrate.

The source of potassium may be a potassium inclusion which contains potassium.

In the solid composition of the present invention, the content of the (C) potassium is preferably 0.2 mass% or more, more preferably 0.6 mass% or more, from the viewpoint of imparting an excellent milk flavor after storage and suppressing occurrence of deteriorated flavor, and is preferably 15 mass% or less, more preferably 8 mass% or less, from the viewpoint of suppressing deterioration of the flavor of the overall solid composition by a salty taste derived from potassium. The content of potassium (C) is preferably 0.2 mass% or more and 15 mass% or less, more preferably 0.6 mass% or more and 8 mass% or less.

In the present specification, analysis of the (C) potassium is performed in accordance with the method described in the following Examples. Incidentally, the content of the (C) potassium includes those derived from the component (A) and other materials such as an additive to be optionally incorporated.

In the solid composition of the present invention, a mass ratio of the component (C) to the component (A), [(C)/(A)], is from 0.2 to 9.0. In the solid composition containing a milk-derived sphingomyelin, in addition to the sugar alcohol, to contain the (C) potassium at a predetermined ratio serves to impart an excellent milk flavor not only the initial timing but also after storage, and to suppress occurrence of deteriorated flavor.

The mass ratio of the component (C) to the component (A), [(C)/(A)], is 0.2 or more, preferably 0.4 or more, more preferably 0.7 or more, from the viewpoint of imparting an excellent milk flavor after storage and suppressing occurrence of deteriorated flavor, and is 9.0 or less, preferably 7.6 or less, more preferably 4.8 or less, from the viewpoint of imparting an excellent milk flavor at an initial timing and after storage and suppressing deterioration of the flavor of the overall solid composition. The mass ratio of the component (C) to the component (A) in the solid composition, [(C)/(A)], is preferably 0.4 or more and 7.6 or less, more preferably 0.7 or more and 4.8 or less.

In addition to the above components (A) to (C), the solid composition of the present invention may appropriately contain, additives such as a mineral (e.g., calcium, magnesium, iron, zinc, chromium, selenium, manganese, molybdenum, copper, iodine, phosphorus, sodium), a vitamin (e.g., vitamin A, vitamin B1, vitamin B2, vitamin B6, vitamin B12, vitamin C, vitamin D, vitamin E, folic acid and salts thereof, or esters thereof), a sweetener (e.g., a monosaccharide, an oligosaccharide, a synthetic sweetener), an acidulant, a perfume, a colorant, a preservative as long as the efficacy of the present invention is not impaired. The content of the additives can be appropriately set adjusted in a range not to impair the objective of the present invention. In addition, a milk-derived component can be used as a raw material for imparting milk flavor. Specifically, it is preferable to use skim milk powder. The content of the skim milk powder is preferably 10 mass% or more, more preferably 20 mass% or more, and further more preferably 25 mass% or more in the solid composition from the viewpoint of imparting excellent milk flavor after storage, and is preferably 60 mass% or less, more preferably 50 mass% or less, and further more preferably 40 mass% or less from the viewpoint of imparting an excellent melting in the mouth. Among the milk-derived ingredients, from the viewpoint of suppressing a bitter taste, an astringent taste, a powdery feeling and the like, a content of casein hydrolysate in the solid composition is preferably 30 mass% or less, more favorably 15 mass% or less, further more favorably 8 mass% or less, further more favorably 5 mass% or less, and even more favorably 3 mass% or less.

The content of water in the solid composition of the present invention is preferably 0.1 mass% or more, more preferably 0.5 mass% or more, and further more preferably 1 mass% or more, from the viewpoint of imparting excellent milk flavor and the texture at the initial timing and after storage. In addition, from the viewpoint of suppressing growth of microorganisms, the content of water is preferably 10.0 mass% or less, more preferably 7.0 mass% or less, and further more preferably 5.0 mass% or less from the viewpoint of handling. The content of water in the solid composition is preferably 0.1 mass% or more and 10.0 mass% or less, more preferably 0.5 mass% or more and 7.0 mass% or less, further more preferably 1 mass% or more and 5.0 mass% or less.

In the present specification, the analysis of the water content is performed in accordance with the method described in the following Examples.

The solid composition of the present invention is not particularly limited as long as it is in a solid state at room temperature (25°C), but specific dosage forms include oral solid formulations such as a capsule, a granule, a powder, a tablet (including a chewable tablet), a pill, and a lozenge. Among them, a granule, a powder, and a tablet are preferable from the viewpoint of being able to be consumed in a small amount at a time and quick intake as a food. These solid compositions may be ingested as they are, but from the viewpoint of quick intake, they are preferably mixed with water, a beverage, or the like to intake.

When such a solid composition is prepared, an acceptable carrier may be appropriately combined as necessary. Examples of the carrier include, an excipient (e.g., lactose, starch (e.g., starch, modified starch, dextrin), microcrystalline cellulose, sucrose, light anhydrous silicic acid, calcium hydrogenphosphate), a binder (e.g., hydroxypropyl methylcellulose, hydroxypropyl cellulose, gelatin, pregelatinized starch,polyvinyl pyrrolidone, polyvinyl alcohol, pullulan, methyl cellulose, hydrogenated oil), a disintegrant (e.g., carmellose, carmellose calcium, croscarmellose sodium, crospopidone, corn starch, low substituted hydroxypropyl cellulose), a lubricant (e.g., calcium stearate, magnesium stearate, sucrose fatty acid ester, stearyl sodium fumarate, talc), a fluidity improver (e.g., silicon dioxide), a taste masking agent (e.g., stevia, aspartame), a flavoring agent, a bulking agent, a surfactant, a dispersing agent, a buffering agent, a coating agent, a diluent, and the like.

The excipient is preferably dextrin from the viewpoint of imparting an excellent flavor after storage. Dextrins assumes a molecular structure in which sugars are polymerized by glycosidic bonds. Examples of the sugar linkage method include α-1,4 linkage, α-1,6 linkage, β-1,2 linkage, β-1,3 linkage, β-1,4 linkage, β-1,6 linkage, and the like, and a single linkage method or two or more linkage methods may be used. DE of dextrin is preferably 5 or more, and more preferably 10 or more, from the viewpoint of improving solubility. From the viewpoint of suppressing browning of the solid composition, it is preferably 30 or less, more preferably 25 or less, and even more preferably 20 or less. Dextrose equivalent (DE) is a ratio of the reducing sugar to the total solid, measured as glucose, which is indicative of the degree of degradation of the starch degradation product. Dextrose equivalent (DE) can be determined by the Wilstetta-Schudel method.

The content of the carrier can be appropriately set within a range that does not impair the object of the present invention. In particular, diluent is preferably from 0.5 to 75 mass%, more preferably from 0.5 to 70 mass%, further more preferably from 5 to 60 mass%, further more preferably from 10 to 56 mass%, further more preferably from 15 to 57 mass% in the solid composition from the viewpoint of improving the milk flavor after storage. From the viewpoint of improving the milk flavor after storage, the content of dextrin is preferably from 1 to 100 mass%, more preferably from 9 to 100 mass%, further more preferably from 15 to 100 mass%, further more preferably from 25 to 100 mass%, and further more preferably from 27 to 100 mass% in diluent.

The solid composition of the present invention may be prepared in accordance with a conventional method, and any appropriate method may be adopted. For example, the components (A) to (C) can be prepared by mixing a carrier and/or an additive, if necessary. The mixing order of the components is not particularly limited, and may be added in any order or may be added at the same time. As the mixing method, an appropriate method such as stirring or shaking can be employed, and a mixing device may be used. The mixture system of the mixing device may be a container rotating type or a container fixing type. As the container rotation type, for example, a horizontal cylindrical type, a V type, a double cone type, a cube type, or the like can be adopted. Further, as the container fixing type, for example, a ribbon type, a screw type, a conical screw type, a paddle type, a fluidized bed type, a Phillips blender, or the like can be employed.

The granulated product may be obtained by a known granulation method. Examples of the granulation method include spray granulation, fluidized bed granulation, compression granulation, tumbling granulation, agitation granulation, extrusion granulation, powder coating granulation, and the like. The granulation conditions can be appropriately selected in accordance with the granulation method. Among them, the fluidized bed granulation method is preferable from the viewpoint of high uniformity of granules and high granulation yield.

When a tablet is used, a mixture of the components (A) to (C), and optionally a carrier and/or an additive may be directly compressed and molded as a raw material powder, or may be granulated by the above-described granulation method, and then the granulated product may be compressed to mold with a tablet molding machine.

When a tablet is produced by pressing the granulated material directly or by pressing the granulated material, commonly used tableting machines may be used such as a rotary tableting machine and a single tableting machine.

The compression-molding pressure at tableting is preferably from 10 to 30 MPa from the viewpoint of maintaining the hardness of the molded product.

Tablet hardness is preferably such that can withstand, for example, transportation, storage, which is preferably from 10 N to 200 N.

The tablet may assume various irregular tablets having a circular or oval shape, an oval shape, a square shape, or the like, but the shape is preferably circular from the viewpoint of ingestibility. For circular tablets, from the viewpoint of ingestibility, the size is preferably from 3 to 30 mm, and more preferably from 3 to 20 mm in diameter. The tablet is preferably given a weight of from 0.1 to 6 g per formulation in terms of convenience and efficacy.

From the viewpoint of imparting excellent good milk flavor at the initial timing and after storage, and suppressing occurrence of deteriorated flavor, the solid composition of the present invention preferably contains the following components (A), (B) and (C):
(A) 0.15 mass% or more and 3.0 mass% or less of a milk-derived sphingomyelin;
(B) a sugar alcohol;
(C) potassium; and
further contains dextrin, and a mass ratio of the component (C) to the component (A), [(C)/(A)], is from 0.2 to 9.0, and a content of dextrin is from 0.5 to 75 mass%.

From the viewpoint of imparting excellent good milk flavor at the initial timing and after storage, ensuring an intake amount to exert an effective physiological effect, and suppressing occurrence of deteriorated flavor, the solid composition of the present invention preferably contains the following components (A), (B) and (C):
(A) 0.2 mass% or more and 2.73 mass% or less of milk-derived sphingomyelin inclusion (as milk-derived sphingomyelin);
(B) sugar alcohol;
(C) potassium;
wherein a mass ratio of the component (C) to the component (A), [(C)/(A)], is from 0.2 to 9.0.

From the viewpoint of imparting excellent good milk flavor at the initial timing and after storage, ensuring an intake amount to exert an effective physiological effect, and suppressing occurrence of deteriorated flavor, suppressing deterioration of the flavor of the overall solid composition, the solid composition of the present invention preferably contains the following (A), (B) and (C):
(A) 0.2 mass% or more and 2.73 mass% or less of milk-derived sphingomyelin inclusion (as milk containing sphingomyelin);
(B) 1 mass% or more and 70 mass% or less of a sugar alcohol selected from the group consisting of maltitol, erythritol and xylitol;
(C) potassium;
wherein a mass ratio of the component (C) to the component (A), [(C)/(A)], is from 0.2 to 7.6.

From the viewpoint of imparting an excellent milk flavor at the initial timing and after storage, ensuring an intake amount to exert an effective physiological effect, suppressing occurrence of deteriorated flavor, and suppressing deterioration of the flavor of the solid composition of the present invention preferably contains the following components (A), (B) and (C):
(A) 0.2 mass% or more and 2.73 mass% or less of milk-derived sphingomyelin inclusion (as milk-derived sphingomyelin);
(B) 1 mass% or more and 70 mass% or less of a sugar alcohol selected from the group consisting of maltitol, erythritol and xylitol;
(C) potassium; and
further contains dextrin, wherein a mass ratio of the component (C) to the component (A), [(C)/(A)], is from 0.2 to 9.0 and a content of the dextrin is from 0.5 to 75 mass%.

From the viewpoint of imparting excellent good milk flavor at the initial timing and after storage, suppressing occurrence of deteriorated flavor, imparting a milk flavor, and imparting excellent melting in the mouth, the solid composition of the present invention preferably contains the following components (A), (B) and (C):
(A) 0.15 mass% or more and 3.0 mass% or lessof milk-derived sphingomyelin inclusion (as milk-derived sphingomyelin);
(B) sugar alcohol;
(C) potassium; and
further contains skim milk powder, wherein a mass ratio of the component (C) to the component (A), [(C)/(A)], is from 0.2 to 9.0, and a content of the skim milk powder is from 10 to 60 mass%.

The solid composition of the present invention has the following components (A), (B) and (C) from the viewpoint of improving milk flavor at an initial timing and after storage, suppressing occurrence of deteriorated flavor, suppressing a bitter taste, an astringent taste, a powdery taste, and the like.
(A) 0.150.11 mass% or more and 3.0 mass% or less of milk-derived sphingomyelin inclusion (as milk-derived sphingomyelin);
(B) sugar alcohol; and
(C) potassium;
wherein a mass ratio of the component (C) to the component (A), [(C)/(A)], is from 0.2 to 9.0 and a content of casein hydrolysate is 30 mass% or less in the composition.

### EXAMPLES

### [Analytical method]

### (1) Analysis of proteins

The content of protein was determined by a combustion method with a nitrogen-protein conversion coefficient of 6.38.

### (2) Analysis of lipid

The content of lipid was determined by an acid decomposition method. To a sample (1 g) was added hydrochloric acid for decomposition. Thereto were added diethyl ether and petroleum ether, and the mixture was stirred. The ether mixture layer was taken out and washed with water. The solvent was evaporated, and the residue was dried. The lipid weight was determined by the measured weight of the dried residue.

### (3) Measurement of water

The content of water was determined by an atmospheric pressure heat drying method (dried at 105°C for 4 hours and then weight was measured).

### (4) Analysis of carbohydrate

The content of carbohydrate was determined by subtracting the contents of protein, lipid, ash and water in the sample, from the total sample mass.

Incidentally, the content of the ash was determined by a direct ashing method (sample was ashed at 550°C, and then weight was measured).

### (5) Analysis of milk-derived sphingomyelin

By using a high-performance liquid chromatograph manufactured by Hitachi, Ltd., a column manufactured by GL Science Co., Ltd. (Inertsil SIL-100A 4.6 mm × 250 mm, 5 µm) was mounted and analysis was carried out at a column temperature of 40°C, and a flow rate 1.2 mL/ of acetonitrile/methanol/water (68/25/7) containing a mobile phase 0.1 v/v% phosphoric acid. The sample volume was 20 µL, and 210 nm absorbance was used to detect milk-derived sphingomyelin. Milk-derived sphingomyelin was determined by using 1,2-dimyristoyl-sn-glycero-3-phosphocholine as an internal control.

### (6) Analysis of Potassium

Measured by atomic absorption spectrophotometry (hydrochloric acid extraction).

### [Raw materials]

Milk-derived sphingomyelin was prepared by centrifuging cream obtained from cow milk, treating the produced buttermilk with a UF membrane, and then spray-drying, and using sphingomyelin inclusion No.1.

The composition of the milk-derived sphingomyelin inclusion No.1 w as: water: 2.4 g/100 g, protein: 51.7 g/100 g, lipid: 28.8 g/100 g, ash: 4.8 g/100 g, carbohydrate: 12.3 g/100 g. Among these, milk-derived sphin gomyelin: 3.79 g/100 g, and potassium: 399 mg/100 g.
Further, the following raw materials were used.
Potassium: Potassium chloride, Kanto Chemical Co., Ltd.
Maltitol: LESYS Fine Powder, Mitsubishi Corporation Life Science Co., Ltd.
Erythritol: Kotobukibussan Co., Ltd.
Xylitol: Xylitol powder, Nippon Garlic Co., Ltd.
Dextrin: Sandec #100, DE value 2 to 5, Sanwa Starch Co., Ltd.

### Examples 1 to 15 and Comparative Examples 1 to 5

Potassium chloride was ground in a mortar. Each raw material component was mixed with the compounding composition shown in Table 1, and the mixture was passed through 20 mesh to obtain a mixed powder.

The "milk flavor" (initial milk flavor) of the obtained mixed powder was evaluated in accordance with the following criteria. The obtained mixed powder (about 20 g) was aluminum-packaged, and it was stored in a constant temperature bath at 55°C for 5 days. After the storage, the "milk flavor" (milk flavor after storage) and "deteriorated flavor" of the stored product were evaluated in accordance with the following criteria. In the evaluation, all samples were first evaluated by three expert panels, where "5" was given to a sample which received the highest rating and "1" was given to a sample which received the lowest rating. The other samples were subsequently positioned relative to the 5-step scale from "1" to "5." Scores were determined through consultation by the three expert panels.

The results are shown in Table 1.

### [Milk flavor]

| | |
|---|---|
| 5: | Milk flavor is strongly felt. |
| 4: | Milk flavor is slightly strongly felt |
| 3: | Milk flavor is weakly felt |
| 2: | Milk flavor is hardly felt |
| 1: | Milk flavor is not felt at all |

### [Deteriorated flavor]

| | |
|---|---|
| 5: | Deteriorated flavor is not felt at all |
| 4: | Deteriorated flavor no astringency is felt |
| 3: | Deteriorated flavor is hardly felt |
| 2: | Deteriorated flavor is felt |
| 1: | Deteriorated flavor is strongly felt |

**Table 1-1**

| (Mass%) | | Comparat ive Example 1 | Exam ple 1 | Exam ple 2 | Exam ple 3 | Exam ple 4 | Exam ple 5 | Exam ple 6 | Comparat ive Example 2 | Comparat ive Example 3 | Exam ple 7 | Exam ple 8 | Exam ple 9 | Exam ple 10 | Comparat ive Example 4 |
|---|---|---|---|---|---|---|---|---|---|---|---|---|---|---|---|
| Formulation | Sphingomy elin inclusion No.1 | 27.8 | 27.8 | 27.8 | 27.8 | 27.8 | 27.8 | 27.8 | 27.8 | 2.8 | 8.3 | 27.8 | 55.6 | 72.0 | 83.3 |
| | (B)Maltitol | 15.0 | 15.0 | 15.0 | 15.0 | 15.0 | 15.0 | 15.0 | 15.0 | 15.0 | 15.0 | 15.0 | 15.0 | 15.0 | 14.4 |
| | Potassium chloride | - | 0.2 | 0.6 | 1.1 | 3.6 | 7.4 | 15.1 | 28.4 | 2.8 | 2.8 | 2.7 | 2.5 | 2.4 | 2.3 |
| | Dextrin | 57.2 | 57.0 | 57.0 | 56.1 | 53.6 | 49.8 | 42.1 | 28.8 | 79.4 | 73.9 | 54.5 | 26.9 | 10.6 | 0.0 |
| | Total | 100.0 | 100.0 | 100.4 | 100.0 | 100.0 | 100.0 | 100.0 | 100.0 | 100.0 | 100.0 | 100.0 | 100.0 | 100.0 | 100.0 |
| Component Composition | (A)Milk-derived sphingomy elin | 1.05 | 1.05 | 1.05 | 1.05 | 1.05 | 1.05 | 1.05 | 1.05 | 0.11 | 0.31 | 1.05 | 2.11 | 2.73 | 3.16 |
| | (C)Potassi um | 0.11 | 0.22 | 0.43 | 0.69 | 2.00 | 3.99 | 8.03 | 15.01 | 1.48 | 1.50 | 1.53 | 1.53 | 1.55 | 1.54 |
| | (C)/ (A) mass ratio | 0.1 | 0.2 | 0.4 | 0.7 | 1.9 | 3.8 | 7.6 | 14.2 | 13.9 | 4.8 | 1.4 | 0.7 | 0.6 | 0.5 |
| | (B)/ (A) mass ratio | 14.2 | 14.2 | 14.2 | 14.2 | 14.2 | 14.2 | 14.2 | 14.2 | 141.3 | 47.7 | 14.2 | 7.1 | 5.5 | 4.6 |
| Evaluation | Initial milk flavor | 4 | 4 | 4 | 4 | 4 | 4 | 3 | 2 | 1 | 3 | 4 | 5 | 4 | 3 |
| | Milk flavor after storage | 3 | 4 | 5 | 5 | 4 | 4 | 3 | 2 | 1 | 3 | 4 | 3 | 3 | 2 |
| | Deteriorate d flavor after storage | 2 | 3 | 4 | 4 | 5 | 5 | 5 | 4 | 5 | 5 | 4 | 3 | 3 | 1 |

**Table 1-2**

| (Mass%) | | Comparative Example 5 | Example 11 | Example 12 | Example 13 | Example 14 | Example 15 |
|---|---|---|---|---|---|---|---|
| Formulation | Sphingomyelin inclusion No.1 | 27.8 | 27.8 | 27.8 | 27.8 | 27.8 | 27.8 |
| | (B)Maltitol | - | 5.0 | 15.0 | 30.0 | 50.0 | 70.0 |
| | Potassium chloride | 1.7 | 1.7 | 1.7 | 1.7 | 1.7 | 1.7 |
| | Dextrin | 70.5 | 65.5 | 55.5 | 40.5 | 20.5 | 0.5 |
| | Total | 100.0 | 100.0 | 100.0 | 100.0 | 100.0 | 100.0 |
| Component C omposition | (A)Milk-derived sphingomyelin | 1.05 | 1.05 | 1.05 | 1.05 | 1.05 | 1.05 |
| | (C)Potassium | 1.00 | 1.00 | 1.00 | 1.00 | 1.00 | 1.00 |
| | (C)/(A) mass ratio | 1.0 | 1.0 | 1.0 | 1.0 | 1.0 | 1.0 |
| | (B)/ (A) mass ratio | - | 4.7 | 14.2 | 28.5 | 47.5 | 66.4 |
| Evaluation | Initial milk flavor | 2 | 4 | 4 | 5 | 5 | 5 |
| | Milk flavor after storage | 2 | 3 | 4 | 5 | 5 | 5 |
| | Deteriorated flavor after storage | 2 | 4 | 4 | 5 | 5 | 5 |

### Examples 16 to 21

Mixed powders were obtained in the same manner as in Example 1 with respect to those obtained by changing the sugar alcohol of component (B) to another (Examples 16 and 17) and those obtained by using sphingomyelin inclusion No.2 prepared by centrifuging cream obtained from cow milk as milk-derived sphingomyelin and spray-drying the produced butter serum (Examples 18 to 21). The "milk flavor" (initial milk flavor), the "milk flavor" (milk flavor after storage) and the "deteriorated flavor" of the obtained mixed powder were evaluated in the same manner as described hereinbefore.

The results are shown in Table 2.

Composition of the milk-derived sphingomyelin inclusion No.2 was: water: 4.0 g/100 g, protein: 29.5 g/100 g, lipid: 11.2 g/100 g, ash: 6.6 g/100 g, carbohydrate: 48.7 g/100 g. Among these, milk-derived sphingomyelin: 1.55 g/100 g, and potassium: 1600 mg/100 g.

**Table 2**

| (Mass%) | | Example 12 | Example 16 | Example 17 | Example 18 | Example 19 | Example 20 | Example 21 |
|---|---|---|---|---|---|---|---|---|
| Formulation | Sphingomyelin inclusion No.1 | 27.8 | 27.8 | 27.8 | - | - | - | - |
| | Sphingomyelin inclusion No.2 | - | - | - | 25.0 | 50.0 | 75.0 | 90.0 |
| | (B) Maltitol | 15.0 | - | - | 15.0 | 15.0 | 15.0 | 9.5 |
| | (B) Erythritol | - | 15.0 | - | - | - | - | - |
| | (B) Xylitol | - | - | 15.0 | - | - | - | - |
| | Potassium chloride | 1.7 | 1.7 | 1.7 | - | - | - | - |
| | Dextrin | 55.5 | 55.5 | 55.5 | 60.0 | 35.0 | 10.0 | 0.5 |
| | Total | 100.0 | 100.0 | 100.0 | 100.0 | 100.0 | 100.0 | 100.0 |
| Component Composition | (A) Milk-derived sphingomyelin | 1.05 | 1.05 | 1.05 | 0.39 | 0.78 | 1.16 | 1.40 |
| | (C) Potassium | 1.00 | 1.00 | 1.00 | 0.40 | 0.80 | 1.20 | 1.44 |
| | (C)/ (A) mass ratio | 1.0 | 1.0 | 1.0 | 1.0 | 1.0 | 1.0 | 1.0 |
| | (B)/ (A) mass ratio | 14.2 | 14.2 | 14.2 | 38.7 | 19.4 | 12.9 | 6.8 |
| Evaluation | Initial milk flavor | 4 | 4 | 4 | 3 | 4 | 5 | 5 |
| | Milk flavor after storage | 4 | 3 | 4 | 3 | 3 | 4 | 5 |
| | Deteriorated flavor after storage | 4 | 3 | 4 | 4 | 4 | 4 | 4 |

As shown in Tables 1 and 2, Examples 1 to 21 containing sugar alcohol and potassium at a predetermined ratio with respect to milk-derived sphingomyelin imparted milk flavor from milk-derived sphingomyelin not only at an initial timing but also after storage, and had little deteriorated flavor.

In contrast, deteriorated flavor was felt in Comparative Example 1 (low ratio of potassium to milk-derived sphingomyelin, though containing sugar alcohol), in Comparative Example 4 (high content of milk-derived sphingomyelin), and in Comparative Example 5 (not containing sugar alcohol). Milk flavor from milk-derived sphingomyelin at the initial timing and after storage was weak in Comparative Example 2 (high ratio of potassium to milk-derived sphingomyelin), and in Comparative Example 3 (low content of milk-derived sphingomyelin), and in Comparative Example 5 (not containing sugar alcohol).

## Claims

1. A solid composition comprising the following components (A), (B) and (C) :
(A) 0.15 mass% or more and 3.0 mass% or less of a milk-derived sphingomyelin;
(B) a sugar alcohol; and
(C) potassium;
wherein a mass ratio of the component (C) to the component (A), [(C)/(A)], is from 0.2 to 9.0.

2. The solid composition according to claim 1, wherein the mass ratio of the component (C) to the component (A), [(C)/(A)], is from 0.2 to 7.6.

3. The solid composition according to claim 1 or 2, comprising milk-derived sphingomyelin inclusion as component (A) in a content of 0.2 mass% or more and 2.73 mass% or less as milk-derived sphingomyelin.

4. The solid composition according to any one of claims 1 to 3, wherein the (B) sugar alcohol is one or more selected from the group consisting of maltitol, erythritol, and xylitol.

5. The solid composition according to any one of claims 1 to 4, wherein a content of the (B) sugar alcohol is 1 mass% or more and 70 mass% or less.

6. The solid composition according to any one of claims 1 to 5, wherein a mass ratio of the component (B) to the component (A), [(B)/ (A)], is from 5 to 70.

7. The solid composition according to any one of claims 1 to 6, wherein the milk-derived sphingomyelin is a cow milk-derived sphingomyelin.

8. The solid composition according to any one of claims 1 to 7, wherein a content of the (C) potassium is from 0.2 to 15 mass%.

9. The solid composition according to any one of claims 1 to 8, further comprising dextrin.

10. The solid composition according to claim 9, wherein the content of the dextrin in the solid composition is from 0.5 to 75 mass%.

11. The solid composition according to any one of claims 1 to 10, further comprising skim milk powder.

12. The solid composition according to claim 11, wherein a content of the skim milk powder in the solid composition is from 10 to 60 mass%.

13. The solid composition according to any one of claims 1 to 12, further comprising casein hydrolysate.

14. The solid composition according to claim 13, wherein a content of the casein hydrolysate in the solid composition is 30 mass% or less.

15. Use of the solid composition according to any one of claims 1 to 14 for mixing with water or beverage to intake.

## Patentansprüche

1. Feste Zusammensetzung, umfassend die folgenden Komponenten (A), (B) und (C):
(A) 0,15 Massen-% oder mehr und 3,0 Massen-% oder weniger eines aus Milch gewonnenen Sphingomyelins;
(B) einen Zuckeralkohol; und
(C) Kalium;
wobei ein Massenverhältnis von der Komponente (C) zu der Komponente (A), [(C)/(A)], 0,2 bis 9,0 beträgt.

2. Feste Zusammensetzung gemäß Anspruch 1, wobei das Massenverhältnis von der Komponente (C) zu der Komponente (A), [(C)/(A)], 0,2 bis 7,6 beträgt.

3. Feste Zusammensetzung gemäß Anspruch 1 oder 2, umfassend eine Einlagerung von aus Milch gewonnenem Sphingomyelin als Komponente (A) zu einem Gehalt von 0,2 Massen-% oder mehr und 2,73 Massen-% oder weniger als aus Milch gewonnenes Sphingomyelin.

4. Feste Zusammensetzung gemäß einem der Ansprüche 1 bis 3, wobei der Zuckeralkohol (B) einer oder mehrere, ausgewählt aus der Gruppe, bestehend aus Maltit, Erythrit und Xylit, ist.

5. Feste Zusammensetzung gemäß einem der Ansprüche 1 bis 4, wobei ein Gehalt an Zuckeralkohol (B) 1 Massen-% oder mehr und 70 Massen-% oder weniger beträgt.

6. Feste Zusammensetzung gemäß einem der Ansprüche 1 bis 5, wobei ein Massenverhältnis von der Komponente (B) zu der Komponente (A), [(B)/(A)], 5 bis 70 beträgt.

7. Feste Zusammensetzung gemäß einem der Ansprüche 1 bis 6, wobei das aus Milch gewonnene Sphingomyelin ein aus Kuhmilch gewonnenes Sphingomyelin ist.

8. Feste Zusammensetzung gemäß einem der Ansprüche 1 bis 7, wobei ein Gehalt an Kalium (C) 0,2 bis 15 Massen-% beträgt.

9. Feste Zusammensetzung gemäß einem der Ansprüche 1 bis 8, ferner umfassend Dextrin.

10. Feste Zusammensetzung gemäß Anspruch 9, wobei der Gehalt an Dextrin in der festen Zusammensetzung 0,5 bis 75 Massen-% beträgt.

11. Feste Zusammensetzung gemäß einem der Ansprüche 1 bis 10, ferner umfassend Magermilchpulver.

12. Feste Zusammensetzung gemäß Anspruch 11, wobei ein Gehalt an Magermilchpulver in der festen Zusammensetzung 10 bis 60 Massen-% beträgt.

13. Feste Zusammensetzung gemäß einem der Ansprüche 1 bis 12, ferner umfassend Caseinhydrolysat.

14. Feste Zusammensetzung gemäß Anspruch 13, wobei ein Gehalt an Caseinhydrolysat in der festen Zusammensetzung 30 Massen-% oder weniger beträgt.

15. Verwendung der festen Zusammensetzung gemäß einem der Ansprüche 1 bis 14 zum Mischen mit Wasser oder einem Getränk zur Einnahme.

## Revendications

1. Composition solide comprenant les composants (A), (B) et (C) suivants :
(A) 0,15 % en masse ou plus et 3,0 % en masse ou moins d'une sphingomyéline dérivée du lait ;
(B) un alcool de sucre ; et
(C) du potassium ;
dans laquelle un rapport en masse du composant (C) au composant (A), [(C)/(A)], va de 0,2 à 9,0.

2. Composition solide selon la revendication 1, dans laquelle le rapport en masse du composant (C) au composant (A), [(C)/(A)], va de 0,2 à 7,6.

3. Composition solide selon la revendication 1 ou la revendication 2, comprenant une inclusion de sphingomyéline dérivée du lait en tant que composant (A) selon une teneur de 0,2 % en masse ou plus et de 2,73 % en masse ou moins en tant que sphingomyéline dérivée du lait.

4. Composition solide selon l'une quelconque des revendications 1 à 3, dans laquelle l'alcool de sucre (B) est un ou plusieurs sélectionnés à partir du groupe constitué du maltitol, de l'érythritol et du xylitol.

5. Composition solide selon l'une quelconque des revendications 1 à 4, dans laquelle une teneur en alcool de sucre (B) est de 1 % en masse ou plus et de 70 % en masse ou moins.

6. Composition solide selon l'une quelconque des revendications 1 à 5, dans laquelle un rapport en masse du composant (B) au composant (A), [(B)/(A)], va de 5 à 70.

7. Composition solide selon l'une quelconque des revendications 1 à 6, dans laquelle la sphingomyéline dérivée du lait est une sphingomyéline dérivée du lait de vache.

8. Composition solide selon l'une quelconque des revendications 1 à 7, dans laquelle une teneur en potassium (C) va de 0,2 à 15 % en masse.

9. Composition solide selon l'une quelconque des revendications 1 à 8, comprenant en outre de la dextrine.

10. Composition solide selon la revendication 9, dans laquelle la teneur en dextrine dans la composition solide est de 0,5 à 75 % en masse.

11. Composition solide selon l'une quelconque des revendications 1 à 10, comprenant en outre du lait écrémé en poudre.

12. Composition solide selon la revendication 11, dans laquelle une teneur en lait écrémé en poudre dans la composition solide va de 10 à 60 % en masse.

13. Composition solide selon l'une quelconque des revendications 1 à 12, comprenant en outre de l'hydrolysat de caséine.

14. Composition solide selon la revendication 13, dans laquelle une teneur en hydrolysat de caséine dans la composition solide est de 30 % en masse ou moins.

15. Utilisation de la composition solide selon l'une quelconque des revendications 1 à 14 pour un mélange avec de l'eau ou une boisson afin d'être consommée.
